# EUROPEAN PATENT APPLICATION

(11) **EP 2 002 847 A1**
(43) Date of publication of application: **17.12.2008**
(21) Application number: 07110111.7
(22) Date of filing: 12.06.2007
(51) Int. Cl.: A61K 47/48, A61L 27/34, A61L 27/54, A61L 31/10, A61L 31/16

(54) **Drug-releasing polyelectrolyte coating, method for forming a drug-releasing polyelectrolyte coating, and implantable device**

(71) Applicant: Capsulution Nanoscience AG, 12489 Berlin (DE)
(72) Inventor: Kröhne, Lutz, 10439, Berlin (DE); Fehring, Volker, 12435, Berlin (DE); Voigt, Andreas, 12621, Berlin (DE)
(74) Representative: Hornung, Jan

(57) **Abstract**

A drug-releasing polyelectrolyte coating comprises:
(a) at least two oppositely charged polyelectrolyte layers;
(b) at least one pharmaceutical active drug which is covalently coupled to polyelectrolytes of at least one of the polyelectrolyte layers;
(c) wherein the drug exhibits a release rate from the polyelectrolyte coating of less than 50% in 3 days.

## Description

This specification describes embodiments pertaining to a drug-releasing polyelectrolyte coating, a method for forming thereof and an implantable device comprising a drug-releasing polyelectrolyte coating.

### BACKGROUND OF THE INVENTION

Recently, bioactive coatings comprising polyelectrolyte multilayers have been studied to evaluate their capability as drug carriers. Bioactive multilayers are also used as a coating for medical devices and also as drug delivery devices. In those systems, the drug is typically embedded in a polyelectrolyte matrix. For delivery of therapeutics, approaches using drugs which are linked to polymers have also been studied.

### SUMMARY OF THE INVENTION

According to an embodiment, a drug-releasing polyelectrolyte coating is provided. The drug-releasing polyelectrolyte coating comprises:
(a) at least two oppositely charged polyelectrolyte layers;
(b) at least one pharmaceutical active drug which is covalently coupled or bound to polyelectrolytes of at least one of the polyelectrolyte layers;
(c) wherein the drug exhibits a release rate from the polyelectrolyte coating of less than 50% in 3 days.

According to another embodiment, a method for forming a drug-releasing polyelectrolyte coating is provided. The method comprises the steps of:
(a) providing a substrate comprising a surface;

(a) depositing at least two oppositely charged polyelectrolyte layers on at least a portion of the surface to form a polyelectrolyte multilayer on the surface, at least one of the polyelectrolyte layers comprising at least one pharmaceutical active drug which is covalently coupled or bound to polyelectrolytes of this layer;
(b) wherein the pharmaceutical active drug is coupled or bound to the polyelectrolyte such that it exhibits a release rate from the polyelectrolyte coating of less than 50% in 3 days.

According to another embodiment an implantable device comprising a drug-releasing coating is provided.

According to another embodiment, a method for forming a drug-releasing polyelectrolyte coating is provided. The method comprises the steps of:
(a) providing a substrate comprising a surface;

(a) depositing at least two oppositely charged polyelectrolyte layers on at least a portion of the surface to form a polyelectrolyte multilayer on the surface, at least one of the polyelectrolyte layers comprising at least one pharmaceutical active drug which is covalently coupled or bound to a polyelectrolyte of this layer;
(b) wherein the drug-coupled polyelectrolyte is deposited on the surface in a hypotonic solution.

The at least two polyelectrolyte layers form a polyelectrolyte coating, which comprises at least one pharmaceutical active drug. Each of the polyelectrolyte layers is comprised of polyelectrolytes having a given charge. The polyelectrolytes of a particular polyelectrolyte layer form a polyelectrolyte network or a polyelectrolyte backbone and can comprise polyelectrolytes of one or more polyelectrolyte material of a given charge. The polyelectrolyte layers attract each other electrostatically. Other attractive forces are based on hydrophobic, van der Walls, and hydrogen bonding interactions. The pharmaceutical active drug is covalently coupled or bound to the polyelectrolytes of at least one of the polyelectrolyte layers such that it exhibits a release rate from the polyelectrolyte coating of less than 50% in 3 days. This allows for a controlled release of the pharmaceutical active drug to provide a prolonged release of the pharmaceutical active drug. The drug-loaded polyelectrolyte coating is therefore particularly suitable for implantable devices for which a release of a pharmaceutical active drug over a long time is desired.

In the context of this specification the release rate or kinetics of the pharmaceutical active drug is determined using a physiological test solution under sink conditions. The physiological test solution is isotonic, wherein isotonic means isotonic with respect to blood. The physiological test solution has a pH-value of 7.4 adjusted by a buffer such as PBS (phosphate buffered saline). The physiological test solution further comprises 0.05 m/v% of a surfactant such as Polysorbat 20 (Polyoxyethylene (20) sorbitan monolaurate) or a solubility agent such as 1 vol.% of a cyclodextrin. It further contains 0.01 M phosphate buffer, 0.0027 M potassium chloride, 0.14 M sodium chloride, 0.05 m/v % Polysorbat 20. The measurement of the release rate is carried out at 37°C under sink condition. Sink condition means that the volume of the test solution is chosen such that the total amount of the drug in the polyelectrolyte coating under test would give a concentration of not more than 10% of the saturation concentration of the drug in this test solution, when the drug is completely released from the polyelectrolyte coating. This ensures that the measurement of the release rate is substantially not affected by the solubility of the drug. If the drug is poorly water soluble, a physiological test solution comprising 1 m/v% of a cyclodextrin is used to increase the solubility of the drug. This allows reducing the required amount of the test solution which facilitates the measurement of the release rate. The physiological test solution is free of enzymes to avoid enzymatic digestion. Further, the physiological test solution should not result in a decomposition of the polyelectrolyte layers so that, after release of the drug from the polyelectrolyte coating, a polyelectrolyte coating without a drug remains. This again is to avoid interference with other release mechanisms such as decompositions. The purpose of the physiological test solution is to determine the release rate of the drug based on the cleavage of the covalent bond. Typically, the physiological test solution contains water, 0.01 M phosphate buffer, 0.0027 M potassium chloride, 0.14 M sodium chloride, and 0.05 m/v % Polysorbat 20 or 1 m/v.% of a cyclodextrin.

### BRIEF DESCRIPTION OF THE DRAWINGS

A full and enabling disclosure of the present invention, including the best mode thereof, to one of ordinary skill in the art, is set forth more particularly in the remainder of the specification, including reference to the accompanying figures. Therein:

Figure 1 shows the chemical structure of paclitaxel.

Figure 2 illustrates the dicyclohexyl carbodiimide(DCC)-mediated conjugation of poly-I-glutamic acid with paclitaxel.

Figure 3 shows the chemical structure of estradiol.

Figure 4 illustrates the DCC-mediated conjugation of poly(acrylic acid) with estradiol.

Figures 5A and 5B show a schematic of the formation of drug loaded polyelectrolyte multilayer (PEM) with polyelectrolyte drug conjugates on a surface (Figure 5A), and the drug release after cleavage from the polyelectrolytes (Figure 5B).

Figure 6 shows a layer-by-layer coating of quartz cells to analyze the formation of PEM with UV spectroscopy.

Figure 7 shows the UV spectrum of poly-I-glutamic acid-paclitaxel-conjugate. A solution with the same molecular weight and concentration of poly-I-glutamic acid was used as baseline.

Figure 8 shows the UV spectrum of poly(acrylic acid)-estradiol-conjugate. A solution with the same molecular weight and concentration poly(acrylic acid) was used as baseline.

Figure 9 shows the formation of drug loaded PEM with a (poly-I-glutamic acid)-paclitaxel-conjugate analyzed with UV spectroscopy at 230 nm.

Figure 10 shows the formation of drug loaded PEM with a (poly-I-glutamic acid)-paclitaxel-conjugate analyzed with QCM-D.

Figure 11 shows the relative drug release of paclitaxel at pH 7.4 analyzed with UV spectroscopy at 230 nm.

Figure 12 shows the absolute amount of released paclitaxel.

Figure 13 shows the formation of drug loaded PEM with poly(acrylic acid)-estradiol-conjugate analyzed with UV spectroscopy at 288 nm.

Figure 14 shows the formation of drug loaded PEM with poly(acrylic acid)-estradiol-conjugate analyzed with QCM-D.

Figure 15 shows the drug release of estradiol at pH 7.4 analyzed with UV spectroscopy at 288 nm.

### DESCRIPTION OF THE PREFERRED EMBODIMENT

For purpose of promoting an understanding of the principles of the invention, reference will now be made to the preferred embodiment illustrated in the drawings and specific language will be used to describe the same. It will nevertheless be understood that no limitation of the scope of the invention is thereby intended, such alterations and further modifications in the illustrated device and/or method, and such further applications of the principles of the invention as illustrated therein being contemplated as would normally occur now or in future to one skilled in the art to which the invention relates.

In the context of the present specification the term "pharmaceutical active drug", which is referred to in remainder of this specification as drug, includes compounds or entities which alter, inhibit, activate or otherwise affect biological events. For example, the drug includes, but is not limited to, anti-cancer substances, anti-inflammatory agents, immunosuppressants, modulators of cell-extracellular matrix interaction including cell growth inhibitors, anticoagulants, antrithrombotic agents, enzyme inhibitors, analgetic, antiproliferative agents, antimycotic substances, cytostatic substances, growth factors, hormones, steroids, non-steroidal substances, and anti-histamines. Examples of indication groups are, without being limited thereto analgetic, antiproliferativ, antithrombotic, anti-inflammatory, antimycotic, antibiotic, cytostatic, immunosuppressive substances as well as growth factors, hormones, glucocorticoids, steroids, non-steroidal substances, genetically or metabolically active substances for silencing and transfection, antibodies, peptides, receptors, ligands, and any pharmaceutical acceptable derivative thereof. Specific examples for above groups are paclitaxel, estradiol, sirolimus, erythromycin, clarithromycin, doxorubicin, irinotecan, gentamycin, dicloxacillin, quinine, morphin, heparin, naproxen, prednisone, dexamethason.

The term "biocompatible" as used in this specification intends to describe material that does not elicit a substantial detrimental response *in vivo*.

The term "biodegradable matrix" as used in this specification intends to describe material which can be decomposed *in vivo.* A biodegradable matrix comprises biocompatible material.

The term "directly covalently coupled" or "directly covalently bound", as used in this specification means that the pharmaceutical active drug is coupled to the polyelectrolytes without any spacer molecule therebetween. This includes, that a functional group of the pharmaceutical active drug reacts with a corresponding functional group of the polymer. For example, the polyelectrolytes of the polyelectrolyte layer are not chemically modified by adding further molecules to the polyelectrolyte after deposition. A skilled person, however, will appreciate that an activation of the functional group of either the polyelectrolyte or the pharmaceutical active drug might be required prior to covalently coupling the pharmaceutical active drug to the polyelectrolyte.

According to certain embodiments, each of the at least two oppositely charged polyelectrolyte layers is comprised of one or more polyelectrolytes of a given charge, so that each polyelectrolyte layer having a specific charge. Adjacent polyelectrolyte layers are formed by different polyelectrolytes so that adjacent polyelectrolyte layers carries opposite charges and attract each other by electrostatic interaction. At least one of the polyelectrolyte layers comprises a drug which is releasably covalently coupled to the polyelectrolytes of that polyelectrolyte layer. The covalently coupling is sufficiently strong to ensure a prolonged and controlled release of the drug from the polyelectrolyte multilayer structure over several days up to several months. This reduces the need for an ongoing administration of the drug and is of high advantage for implantable medical devices which remain in the human or animal body for a long time. The drug release out of the polyelectrolyte multilayer structure is highly localised and thus allows optimisation of the required dose. An example of such a device is a stent or a catheter. The material of the implantable device is typically biologically inert. Another example is a biodegradable matrix which can be decomposed *in vivo.*

In certain embodiments the biodegradable matrix can comprise a polyelectrolyte matrix which is formed by precipitation. The matrix has a thickness which is typically substantially thicker than the thickness of a single polyelectrolyte layer of the polyelectrolyte coating and particularly thicker than the polyelectrolyte coating. A single polyelectrolyte layer may have a thickness of about 1 nm to about 5 nm depending on the coating conditions and the polyelectrolyte material used. The polyelectrolyte coating may have a thickness of about 10 nm to about 100.000 nm (100 µm) depending on the coating conditions, the number of, and the polyelectrolyte material used for, the polyelectrolyte layers. The biodegradable matrix may have a thickness of about 1 µm to about 5000 µm. Typically, contrary to the polyelectrolyte coating, which comprises substantially ordered polyelectrolyte layers, the matrix must not comprise a layer arrangement formed by alternatingly charged polyelectrolyte layers. If the matrix is formed by precipitated polyelectrolytes, the matrix may have the structure of a compact meshwork. Using the same polyelectrolyte material for forming the matrix and the polyelectrolyte coating ensures sufficient biodegradability of the implantable device. Examples of material used for the biodegradable matrix include, but are not limited thereto, calcium phosphate, polyesters, polyanhydrides, polypetides, nucleic acids, hydroxyapatite, polylactides, poly(lactide-co-glucolide), cationic and anionic polyelectrolytes, biopolymers, and mixtures thereof.

In other embodiments, the matrix can have a woven structure suitable to be placed into, or on certain areas of, the human or animal body to cover, for example, internal or external lesions.

It is also possible, that the polyelectrolyte coating is detached from a supporting substrate prior to implanting. In this case, the coating itself is an implantable device.

The polyelectrolyte layer can be formed in a self-assembled manner to produce a polyelectrolyte multilayer structure such as a Layer-by-Layer (LbL) structure. In certain embodiments the polyelectrolyte coating can comprise at least three alternatingly charged polyelectrolyte layers. In other certain embodiments, the polyelectrolyte coating can comprise at least four alternatingly charged polyelectrolyte layers. Other embodiments may comprise 10 or more, such as 20 or more or even 30 or more alternatingly charged polyelectrolyte layers. The number of polyelectrolyte layers is generally not restricted and can be tailored according to specific requirements.

In certain embodiments, the polyelectrolyte layer or layers of a given polarity can be formed by the same polyelectrolyte material or by different polyelectrolyte materials. For example, the same polyelectrolyte material can be used for forming cationic polyelectrolyte layers and different polyelectrolyte materials can be used for forming the anionic polyelectrolyte layers. Further, the same polyelectrolyte material can be used for forming anionic polyelectrolyte layers but different polyelectrolyte materials can be used for forming the cationic polyelectrolyte layers. It is also possible to use different polyelectrolyte material for cationic and anionic polyelectrolyte layers, respectively.

The material of a polyelectrolyte layer can be formed by a single type of polyelectrolytes or a mixture of polyelectrolytes which carries the same charge.

The at least two oppositely, or alternatingly, charged polyelectrolyte layers can be formed by sequential deposition from solutions comprising the respective polyelectrolytes or a polyelectrolyte mixture. The charge of the polyelectrolytes determines the charge of the polyelectrolyte layer to be formed. For deposition of the polyelectrolyte layers and the drug-loaded polyelectrolyte layer or layers, a polyelectrolyte solution can be for instance sprayed onto a substrate on which the polyelectrolyte coating is to be formed. Alternatively, or in combination, the substrate can be immersed or dipped into a polyelectrolyte solution or a polyelectrolyte solution can be poured onto the surface of the substrate. Between the depositions of the polyelectrolyte layers at least one washing or rinsing step in a solution without polyelectrolytes can be performed to remove not-assembled polyelectrolyte material. Alternatively, or in addition to that, an intermediate drying step or steps can be performed according to a coating protocol. After a drying step, the same polyelectrolyte solution as used immediately before the drying step can be deposited again on the surface since the drying can lead to a partial exposure of binding sides or charges of the underlying polyelectrolyte layer. By applying such a sequence, the load of a particular polyelectrolyte, and hence of a drug, can be further increased.

To improve the stability of the polyelectrolyte coating the at least two oppositely, or alternatingly, charged polyelectrolyte layers can be additionally covalently coupled to each other. It is further possible to provide an additional coating onto the polyelectrolyte coating such as lipid bilayers and membranes.

The polyelectrolyte material can also be biodegradable so that the polyelectrolyte coating is decomposed *in vivo.*

In certain embodiments, at least one of the polyelectrolyte layers comprises a drug. The drug is covalently coupled to the polyelectrolyte material that forms this polyelectrolyte layer. A polyelectrolyte layer comprising a drug is referred to as drug-conjugated polyelectrolyte layer. In other certain embodiments, the polyelectrolyte coating comprises at least two drug-conjugated polyelectrolyte layers. In further embodiments, the polyelectrolyte coating comprises at least three drug-conjugated polyelectrolyte layers such as five or ten drug-conjugated polyelectrolyte layers. For example, drug-conjugated polyelectrolyte layers may alternate with nonconjugated polyelectrolyte layers, so that, for example, every second polyelectrolyte layer is drug-conjugated. By increasing the number of the drug-conjugated polyelectrolyte layers the drug loading of the polyelectrolyte coating can be increased and tailored. Suitable polyelectrolyte materials for covalently coupling to the drug may include, but not limited thereto, polycarboxylic acid and polyamines.

In certain embodiments, the polyelectrolyte coating comprises at least two different drugs which are covalently coupled to the same or to different polyelectrolyte layers. For example at least one of the at least two drugs is coupled to one polyelectrolyte layer and the other drug is coupled to two or more other polyelectrolyte layers.

The at least one drug is covalently coupled to the polyelectrolytes for providing a given stability of the coupling between the drug and the polyelectrolytes. The coupling should be stable enough so that the polyelectrolyte coating exhibits a drug release rate of less than 50% in 3 days. In certain embodiments, the drug release rate is less than 50% in 5 days. In other embodiments, the drug release rate is less than 50% in 7 days, or even less than 50% in 10 or 14 days. Hence, the drug can be administered over a long period which is of great advantage particularly for implanted devices so that they have a slow drug release rate. The covalent link or linkage between the drug and the polyelectrolytes should be chosen such that the desired release rate can be obtained.

On the other hand, certain embodiments includes a polyelectrolyte coating having a drug release rate of up to 90% in 12 month, particularly of up to 90% in 9 or even 6 month.

In certain embodiments, the at least one drug is covalently coupled by at least one specific link formed directly between the drug and the polyelectrolytes. The link can be selected from the group containing esters, amides, carbamides, disulfides, and azomethines. These links provide sufficient stability to ensure a controlled or retarded and prolonged drug release. The link can be formed by a condensation reaction. Azomethine linkages are typically more stable than for example ester linkages which can be hydrolysed. Since the drug shall be released from the polyelectrolyte coating, linkages are of interest which provides a releasable link between the drug and the polyelectrolytes. Further, since the release of the drug shall take place *in vivo*, releasability means with respect to *in vivo* conditions.

The polyelectrolytes comprise at least one specific functional group which allows covalently coupling of the drug. Accordingly, the drug also comprises at least one specific functional group corresponding to the functional group of the polyelectrolytes. Typically, the polyelectrolytes comprise a plurality of functional groups to allow coupling of a plurality of drug molecules to increase drug loading.

In certain embodiments, the polyelectrolytes are polymers comprising a plurality of coupled monomers. Each monomer may comprise at least one functional group.

The at least one drug can be coupled to one or more polyelectrolyte layers of the polyelectrolyte coating by different specific links to tailor the release rate. For example, drug molecules can be coupled to polyelectrolyte layers, which are arranged proximate to a surface on which the polyelectrolyte coating is formed, by a given link while other drug molecules can be coupled to polyelectrolyte layers, which are arranged distal to the surface, by another specific link. Different drugs can be coupled by different or by the same specific link to the polyelectrolytes.

In addition to the specific link used for coupling the drug to the polyelectrolytes, the numbers of the polyelectrolyte layers and the arrangement of the drug-conjugated layer or the drug-conjugated layers within the polyelectrolyte coating may influence the drug release rate. By arranging a drug-conjugated polyelectrolyte layer proximate to a surface, on which the coating is formed, and by arranging a plurality of unconjugated polyelectrolyte layers on the drug-conjugated polyelectrolyte layer the release rate might be further reduced. Without wishing to be bound, it is believed that the local physicochemical environment is partially altered within the polyelectrolyte layer system which might affect the ability to release the drug. The water content of a typical polyelectrolyte multilayer structure is of the order of about 50 %. The polarity and other solvent/water parameters are influenced by the interaction of the high polymer segment and/or drug densities with the adjacent restricted water environment. It is, however, assumed that the type of the covalent link between the drug and the polymer mainly determines the release rate. When considering *in vivo* conditions, the release rate might be reduced in comparison with the release rate observed in the physiological test solution due to the influence of enzymes such as esterase and amidase as well as of biosurfactants. To reduce the influence of enzymes, enzyme-inhibitors may also be incorporated in the polyelectrolyte coating to maintain slow drug release.

In certain embodiments the at least one drug is soluble (at least about 33 mg/ml) or at least freely soluble (at least about 100 mg/ml) in water. In other certain embodiments, the at least one drug is sparingly soluble (less than about 33 mg/ml), slightly soluble (less than about 10 mg/ml), very slightly soluble (less than about 1 mg/ml) or even insoluble (less than about 0.1 mg/ml). For drugs which are only sparingly soluble or even less soluble the polyelectrolyte coating provides a drug carrier for an aqueous environment such as *in vivo* environment. For at least soluble drugs the release rate can effectively be reduced in comparison with drug delivery system in which the drug is only embedded in a matrix so that a slow release rate can be obtained also for at least soluble drugs. The release rate is therefore substantially independent of the solubility of the drug unlike other drug delivery systems, which comprise drugs embedded in a matrix and covered by a diffusion barrier layer. The release rate of the latter systems is governed by the solubility of the drug in the external solution.

In certain embodiments, the drug-conjugated polyelectrolyte layer is deposited on a surface of a substrate in a hypotonic solution. It has been observed that deposition of drug-conjugated polyelectrolyte in hypotonic solutions, unlike common experience, results in a high loading of the polyelectrolyte layer. The hypotonic solution may have a salt molarity of about 0.1 M or less or even of about 0.05 M or less. High deposition-load has also been observed in salt-free solutions.

Without wishing to be tied to theory, it is believed that a low salt concentration - contrary to previous approaches using a solution comprising at least 0.2 M salt to reduce electrostatic depletion of like-charged polyelectrolytes during deposition - enables the polyelectrolyte/substrate system to get into more intimate contact. The interplay between electrostatic and hydrophobic interactions is changed to such an extent that the adsorbed amount per surface area is increased leading to a higher loading of the polyelectrolyte and hence the drug in the polyelectrolyte coating.

The deposition of the polyelectrolytes can also be influenced by the interaction between the charged polyelectrolytes and the charged surface onto which the polyelectrolytes are to be deposited and the interaction between the charged polyelectrolytes among each other. These interactions can be at least partially controlled by the ion strength of the solution. Therefore, the ion strength of the solution is adjusted in certain embodiments to increase the amount of the deposited polyelectrolytes.

Alternatively, or in addition to, intermediate drying steps may help to increase the deposition rate. Typically, a polyelectrolyte solution is brought into contact with the surface and the surface is allowed to dry for a given time, for examples 10 to 60 sec by purging with Nitrogen gas. Subsequently, the same polyelectrolyte solution is again brought into contact with the surface. It has been observed that intermediate drying results in an increased load of polyelectrolytes and drug-conjugated polyelectrolytes.

The polyelectrolyte coating and the implantable devices described in this specification can be easily sterilised and stored without detrimental effects to the drug and its release properties. Further, the polyelectrolyte coating can be modified according to specific needs and comply with pharmaceutical regulations.

Subsequently, specific embodiments are described which comprise the formation of a polyelectrolyte multilayer on a surface of an implantable medical device such as a stent, a catheter, a pacemaker, an artificial vessel of permanent or transient types, or a permanent or transient implant. The polyelectrolyte multilayer is a drug eluting polyelectrolyte multilayer (PEM) with a high drug loading. The drug or drugs is or are covalently coupled to one or several polyelectrolyte layers. The layers to which the drug or the drugs are coupled can comprise the same or different polyelectrolytes. PEM are prepared by using the LbL-process which comprises an alternating deposition of oppositely charged layers with or without linked active compounds or drugs. The LbL process can be carried out with aqueous, aqueous-organic solvent or organic mixtures for forming layered, erasable polymer multilayers formed by hydrogen-bonded sequential self-assembly. The latter approach might be useful for deposing hydrophobic drugs. The alternating cationic polyelectrolyte layer could be for example chitosan, collagen, gelatin, polyallylamine hydrochloride, polyarginine, polydiallyldimethylammoniumchloride, polyethylenimine, polylysine, protamine or other cationic charged polymers. The alternating polyanionic layer can include albumin, alginate, carboxymethylcellulose, carrageenan, chondroitin 6-sulfate, dextransulfate, gelatin, heparin, hyaluronic acid, pectin, polyacrylic acid, polyglutamic acid, polymethacrylic acid, polystyrensulfonic acid, polystyrenesulfonic acid-co-maleic acid, polyvinylsulfonic acid, sulfoethylcellulose and other suitable negatively charged polyelectrolytes.

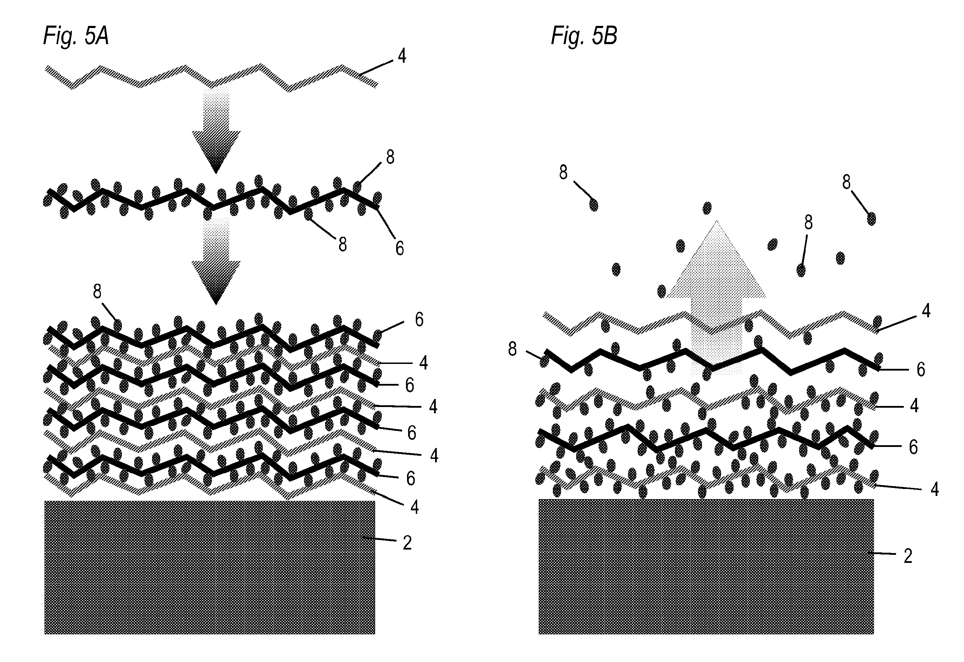

Figure 5A shows the formation of the polyelectrolyte coating by consecutive adsorption of polyelectrolyte layers on a substrate 2. In this embodiment, first an unconjugated polyelectrolyte layer 4 is deposited, followed by the deposition of a drug-conjugated polyelectrolyte layer 6 comprising drug molecules 8. This deposition sequence can be repeated multiple times. Deposition of unconjugated polyelectrolyte layers as a capping layer can be finally performed.

The release of the drug is illustrated in Figure 5B showing in a different build up the cleavage of the drug molecules 8 from the polyelectrolyte layers 4 and 6 which are both conjugated with the drug in this example. Further, a partial or complete slow decomposition of the polyelectrolyte coating can also occur. The polyelectrolytes should be chosen such that the release of the drug is mainly governed by the cleavage of the covalent link.

To ensure a high loading efficiency of the drug and the drug release out of the PEM the polyelectrolyte and the type of the covalent bonding should be selected suitably. After cleavage from the polyelectrolyte the drug can diffuse out of the PEM. The linkage between the drug and the polyelectrolyte strongly influences the drug release. If the polyelectrolyte-drug linkage is sufficiently stable, a slower drug release will be achieved. To adjust the drug release rate different tailor-made polyelectrolyte-drug linkages can be combined within an overall PEM.

According to an embodiment the polyelectrolyte or the polyelectrolytes to which the drug is coupled can be selected according to the following criteria:
(a) The polyelectrolytes need to have suitable functional groups for drug attachment.
(b) The PEM formed by the polyelectrolytes should allow the diffusion of the drug out of the PEM.
(c) The PEM should exhibit sufficient long-term stability with and without the drug.

The coupling chemistry for the conjugation of the drug to polyelectrolytes depends on the functional group or groups which are present in the drug molecule and suitable for a coupling reaction. Such functional groups are amongst others hydroxyl groups, amino groups, carboxyl groups, thiol groups and oxo groups from aldehyd or ketone substructures and can be easily identified from those which are skilled in the art.

Depending on the chemical properties of drug and polymers and the intended release characteristics the coupling between drug molecules and polyelectrolytes can be done either by a direct coupling reaction between drug and polymer or by using a bifunctional chemical substance (linker) which covalently connects drug and polymer. Here especially the formation of esters, amides, carbamides, disulfides, azomethins and other cleavable coupling products with functional groups of the drug is intended.

According to certain embodiments the direct coupling between drug and polymer can either be done by using a pre-activated functional group of the drug and / or polymer or by applying an in situ activation of functional groups of the drug and / or polymer. Such pre-activated functional groups are among others pentafluorophenyl esters, p-nitrophenyl esters, N-hydroxysuccinimide activated esters, sulfo-N-hydroxysuccinimide activated esters and 3-hydroxy-2,3-dihydro-4-oxo-benzo-triazone esters. Among other chemical reactions of different suitable functional groups such in situ activation can for example be done with carboxyl groups by using activating reagents like carbodiimides (e.g." dicyclohexyl carbodiimide - DCC), O-Benzotriazole-N,N,N',N'-tetramethyl-uronium-hexafluorophosphate (HBTU), O-(Benzotriazol-1-yl)-N,N,N',N'-tetramethyluronium tetrafluoroborate (TBTU), Benzotriazole-1-yl-oxy-tris-(dimethylamino)-phosphonium hexafluorophosphate (BOP).

According to certain embodiments the coupling between drug and polymer can be done by using a bifunctional chemical substance (linker) which covalently connects drug and polymer. Such linker can either be homobifunctional or be heterobifunctional. Examples for homobifunctional linkers are amongs others amine reactive linkers (like Lomant's reagent), sulfhydryl reactive linkers (like 1,4-Di-[3'-(2'-pyridyldithio)-propionamido]butane), hydroxy reactive linkers (like dicarbonic acids) and carboxy reactive linkers (like diols). Examples for heterobifunctional linkers are amongs others amine-carboxyl reactive linkers (like 3-[(2-Aminoethyl)dithio]propionic acid), amine-sulfhydryl reactive linkers (like N-Succinimidyl-3-(2-PyridylDithio)-Propionate) and sulfhydryl-hydroxyl reactive linkers (like N-[p-Maleimidophenyl]isocyanate).

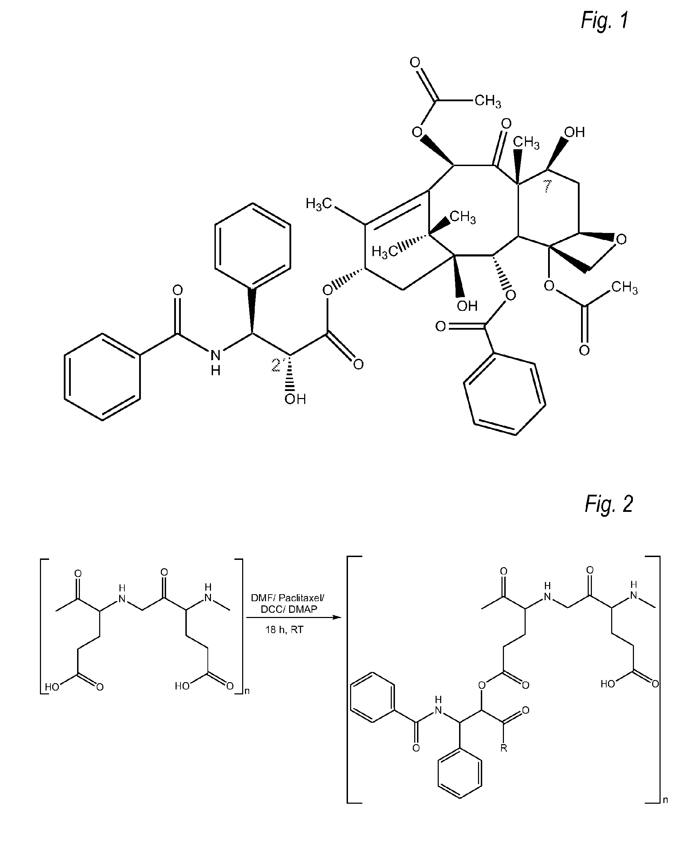

Specific examples comprise the synthesis of different polyelectrolyte-paclitaxel-conjugates with poly-I-glutamic acid as anionic polyelectrolyte. The conjugation can be mediated by DCC. The reaction of DCC with the carboxylate group of the poly-I-glutamic acid and the hydroxyl group of the paclitaxel can be carried out in N,N-dimethylformamide (DMF) to form an ester. The reaction scheme is presented in Figure 2. Poly-I-glutamic acid sodium salt was obtained from Sigma Aldrich with a molecular weight (Mw) of 15.000-50.000 (P4761-1G) and of Mw of 50.000-100.000 (P4886-1 G). Figure 1 shows the chemical structure of paclitaxel.

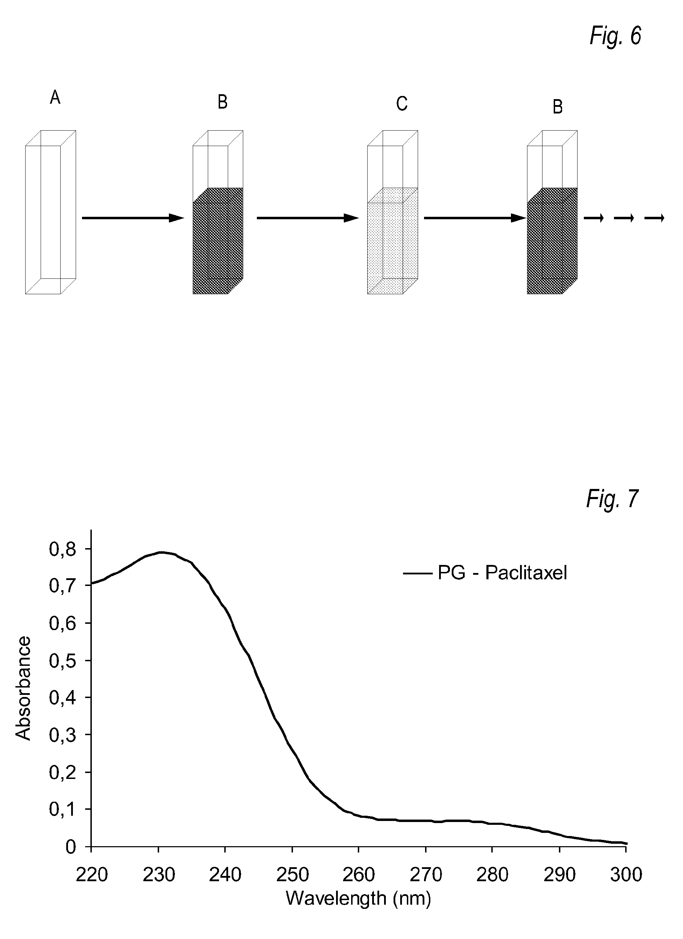

Poly-I-glutamic acid sodium salt (PG-Na) was first converted to Poly-I-glutamic acid in its proton form. The pH of the aqueous solution was adjusted to 2 using 0.2 M HCl. After stirring over 2h at room temperature the precipitate was collected, dialyzed against water and finally lyophilized (3 days at 0.1 mbar at 30°C). To a solution of poly-I-glutamic acid (75 mg) in dry N,N-dimethylformamide was added paclitaxel (22 mg), DCC (15 mg) and dimethylaminopyridin (1-10 mg). The reaction at room temperature was allowed to proceed for 18 hours. Thin layer chromatography showed complete conversation of paclitaxel to polyelectrolyte conjugate. To stop the reaction, the reaction mixture was poured into chloroform (stabilized with amylen). The resulting precipitate was collected and dried in vacuum. To obtain the sodium salt of the poly-I-glutamic-paclitaxel-conjugate the dried product was dissolved in 1.0 M NaHCO₃ and dialyzed against water to remove low molecular weight contaminants. Finally the dialysate was filtrated (0.8 µm) and lyophilized to obtaine a white powder. By changing the weight ratio of paclitaxel to polyglutamic acid in starting materials, polymeric conjugates of various paclitaxel fractions can be synthesized. The payload of paclitaxel is strongly influencing the water solubility of the conjugate. Increased drug loading will decrease the water solubility. A conjugate with a paclitaxel content up to 35% (w/w) should be still soluble in water. The paclitaxel content in the poly-I-glutamic acid paclitaxel conjugate was estimated with UV spectroscopy based on the molar extinction coefficient of paclitaxel (ε_{230 nm} = 29.800). A stock solution of the conjugate (2 g/l in 50 mM PBS pH 6.5) was prepared and diluted to a concentration of 0.2 g/l. The UV absorption at 230 nm was measured. As blank value a freshly prepared solution of poly-I-glutamic acid with the same concentration was used (Figure 7).

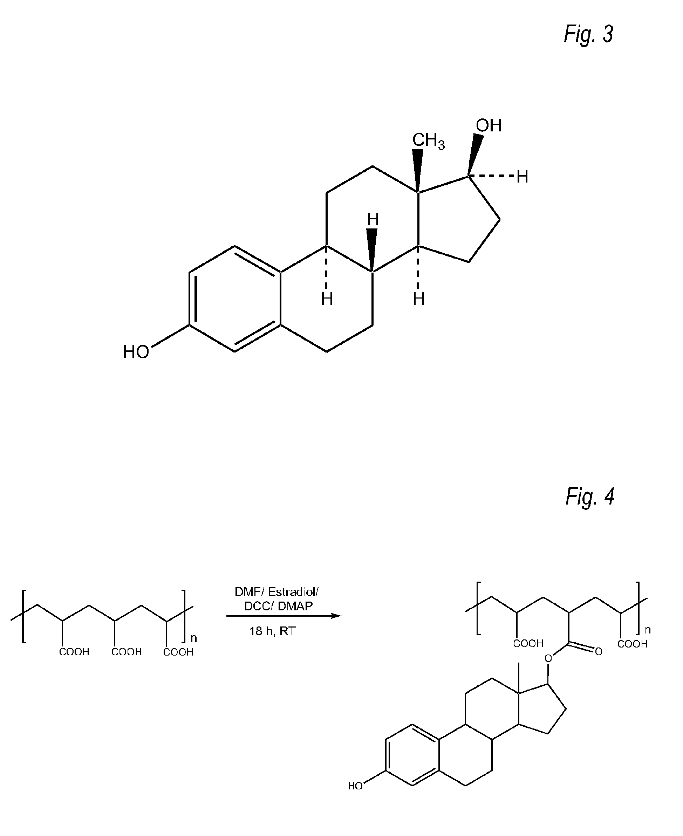

Specific examples comprise the synthesis of polyelectrolyte-estradiol-conjugates with poly(acrylic acid) as anionic polyelectrolyte. Figure 3 shows the chemical structure of estradiol. The conjugation can be mediated by DCC. The reaction of DCC with the carboxylate group of the poly(acrylic acid) and the hydroxyl group of the estradiol can be carried out in N,N-dimethylformamide (DMF) to form an ester. The reaction scheme is presented in Figure 4. Poly(acrylic acid) was obtained from Sigma Aldrich with a molecular weight (Mw) of 100.000.

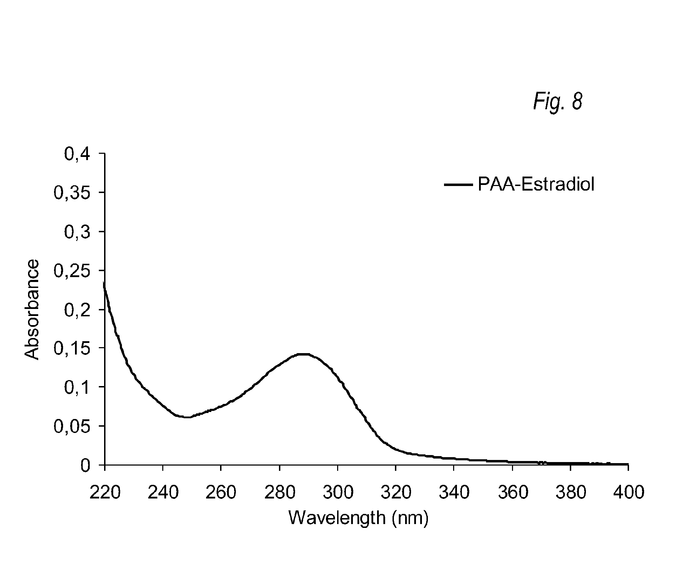

Estradiol was covalently attached to poly(acrylic acid) (PAA) via the formation of an ester bond. To a solution of PAA (100 mg, 1.5 mmol) in dry N,N-dimethylformamide was added estradiol (9 mg), DCC (25 mg) and dimethylaminopyridin (1-10 mg). The reaction was allowed to proceed over night at room temperature. To stop the reaction, the reaction mixture was poured into chloroform (stabilized with amylen). The resulting precipitate was collected and dried in vacuum. To obtain the sodium salt of the conjugate the dried product was dissolved in 1.0 M NaHCO₃ and dialyzed against water to remove low molecular weight contaminants. Finally the solution was filtrated (0.8 µm), lyophilized and a white powder was obtained. By changing the employed weight ratio estradiol / poly(acrylic acid) polymeric conjugates having different estradiol contents can be synthesized. The Estradiol content in poly(acrylic acid)-estradiol-conjugate was analyzed by size exclusion chromatography (SEC). The SEC system consisted of a BioSep SEC-S3000 (300x7.8 mm) column (Phenomenex, Torrance, CA), a Merck Hitachi isocratic LC pump, autosampler and a DAD UV/Vis detector. The eluent (acetonitril/ 10 mM PBS, pH 5.5, 30:70) was running at 1.0 ml/min. We analyzed the chromatogram at 283 nm (conjugated and free estradiol). PAA shows nearly zero absorption at 283 nm. The conjugation of E2 to PAA results an absorption increase at 283. The Estradiol content in the poly(acrylic acid)-estradiol-conjugate was estimated with UV spectroscopy based on the molar extinction coefficient of estradiol (ε_{288 nm} = 2.160). A stock solution of the conjugate (2 g/l in 50 mM PBS pH 6.5) was prepared and diluted to a concentration of 0.1 g/l. The UV absorption at 288 nm was measured. As blank value a freshly prepared solution of poly(acrylic acid) with the same concentration was used (Figure 8).

The PEM was constructed via alternated polyelectrolyte deposition onto different substrates such as glass and quartz cuvettes. Figure 6 shows the formation of a polyelectrolyte coating on the inside of a quartz cuvette to allow confirmation of the Layer-by-Layer formation by UV spectroscopy. For determining the adsorption of the layers the UV absorption was measured after each adsorption step. Before polyelectrolyte adsorption, the glass slides and quartz cuvettes were cleaned with H₂O₂/ NH₃/ H₂O (1:1:5) and extensively rinsed with water. All substrates were coated with a precursor layer of polyethylenimine (PEI) (A in Figure 6). Between each deposition step (B, C in Figure 6), the films were four times rinsed with water. The layer-by-layer build-up was followed by UV spectroscopy and quartz crystal microbalance with dissipation (QCM-D). For the preparation of drug eluting PEM the polyelectrolyte drug conjugates listed in Table 1 were used as coating materials.

**Table 1: Polyelectrolyte drug conjugates for build-up of polyelectrolyte multilayers**

| Abbreviation | Polyelectrolyte | Molecular weight of the polyelectrolyte [kDa] | Molar ratio of paclitaxel to poly-I-glutamic acid |
|---|---|---|---|
| PAA-E2 | poly(acrylic acid) | 100 | 1:13 |
| PG30-Ptx | poly-I-glutamic acid | 15-50 | 1:23 |
| PG75-Ptx | poly-I-glutamic acid | 50-100 | 1:30 |

The release rate was determined by rinsing the cuvettes with a sufficient volume of a physiological test solution containing water, a buffer and 0.05 vol.% Polysorbat 20. No additional cyclodextrin was required since the release rate was determined based on the amount of the drug which remained in the polyelectrolyte coating and not on the amount of the drug released into the physiological test solution. The amount of the drug in the polyelectrolyte coating was determined using UV-Spectroscopy.

### Example 1:

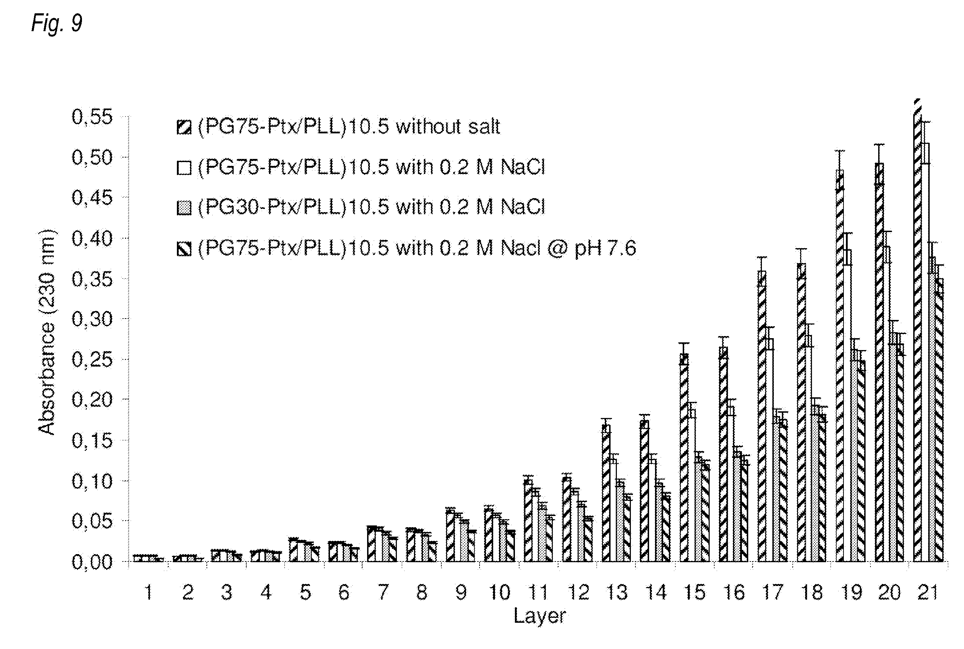

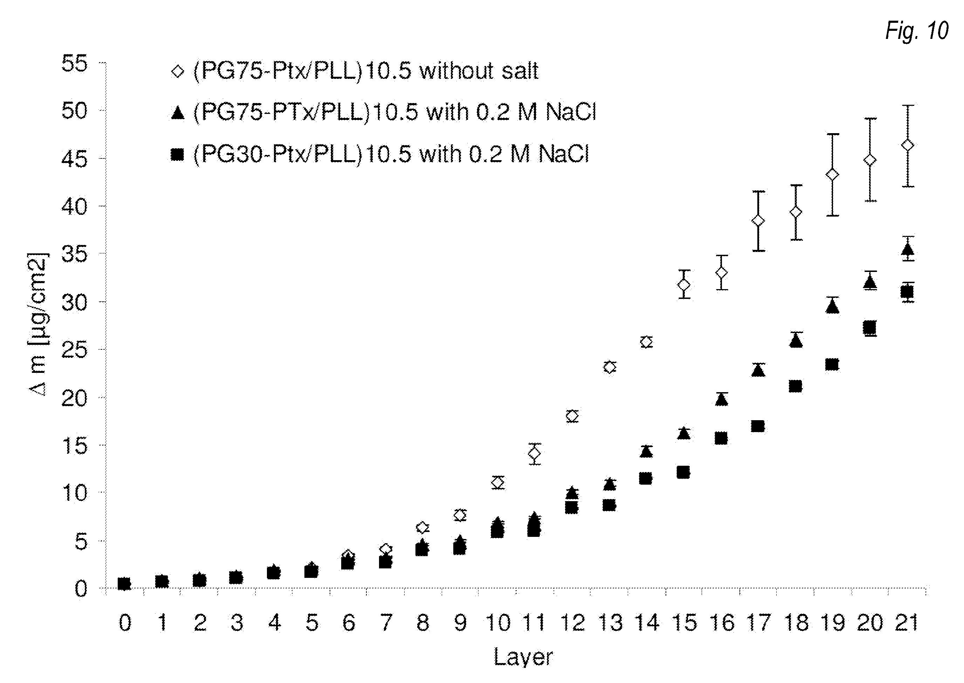

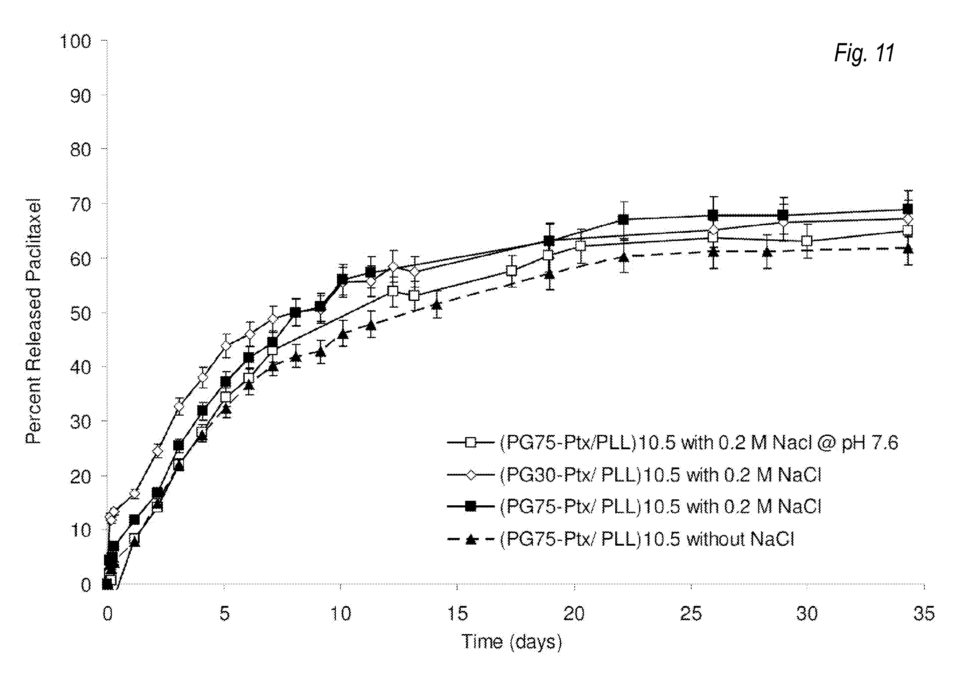

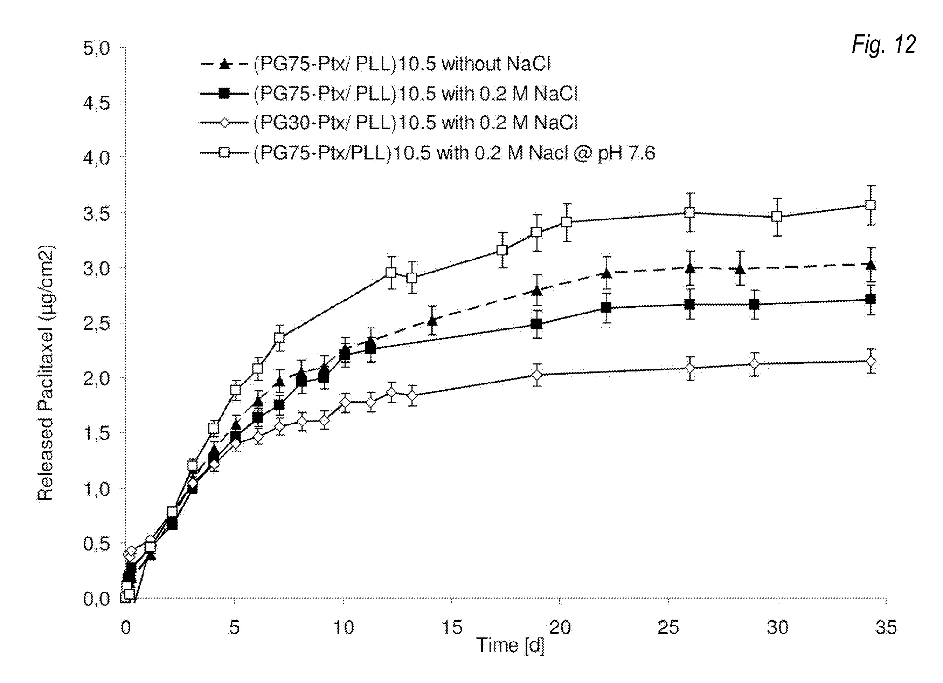

PEM were constructed with PG75-Ptx and PLL (poly-L-lysine) at pH 5.6 (50 mM acetate buffer solution), 0.2 mg/ml polyelectrolyte concentration without additional salt. The build-up was monitored with UV spectroscopy (Figure 9) and QCM-D (Figure 10) up to 21 polyelectrolyte layers. Under this condition we found the pronounced film growth for this polyelectrolyte combination. The coating exhibits an exponential growth. Figure 11 shows the relative drug release while Figure 12 shows the absolute amount of released drug.

### Example 2:

PEM were constructed with PG75-Ptx and PLL at pH 5.6 (50 mM acetate buffer solution), 0.2 mg/ml polyelectrolyte concentration with 0.2 M NaCl. The build-up was monitored with UV spectroscopy (Figure 9) and QCM-D (Figure 10) up to 21 polyelectrolyte layers. Compared to the build-up of PEM without additional salt a lower film growth was observed. The coating exhibits an exponential growth. The drug release is displayed in Figures 11 and 12.

### Example 3:

PEM were constructed with PG30-Ptx and PLL at pH 5.6 (50 mM acetate buffer solution), 0.2 mg/ml polyelectrolyte concentration with 0.2 M NaCl. The build-up was monitored with UV spectroscopy (Figure 9) and QCM-D (Figure 10) up to 21 polyelectrolyte layers. Compared to the build-up of PEM without additional salt a lower film growth was observed. The coating exhibits an exponential growth. The drug release is displayed in Figures 11 and 12.

As becomes evident from Figures 9 and 10, the polyelectrolyte coating deposited in salt-free solution has a higher drug load than the polyelectrolyte coatings deposited in a 0.2 M NaCl solution. The comparison between examples 1 to 3 show that the absolute amount of released paclitaxel from polyelectrolyte coatings formed in salt-free solutions is than higher than from polyelectrolyte coatings deposited in 0.2 M NaCl solution (Figure 12) while the relative release is substantially the same (Figure 11).

### Example 4:

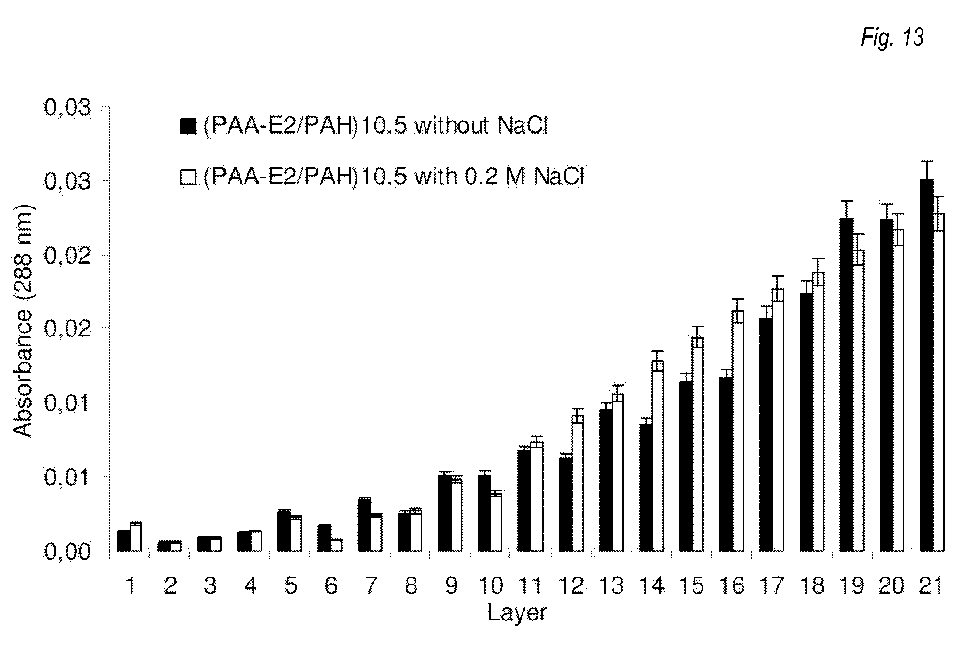

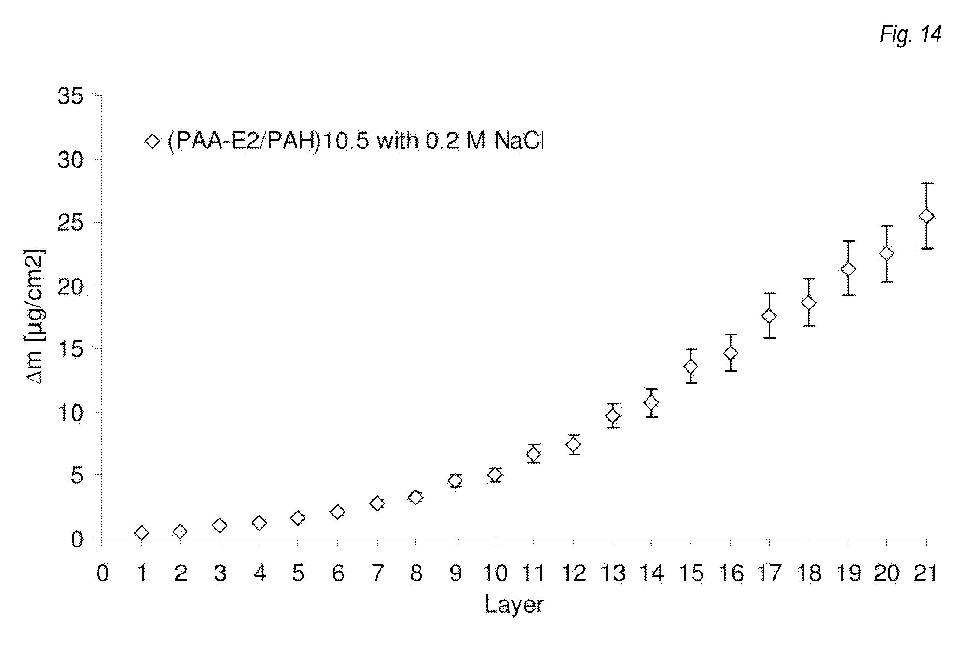

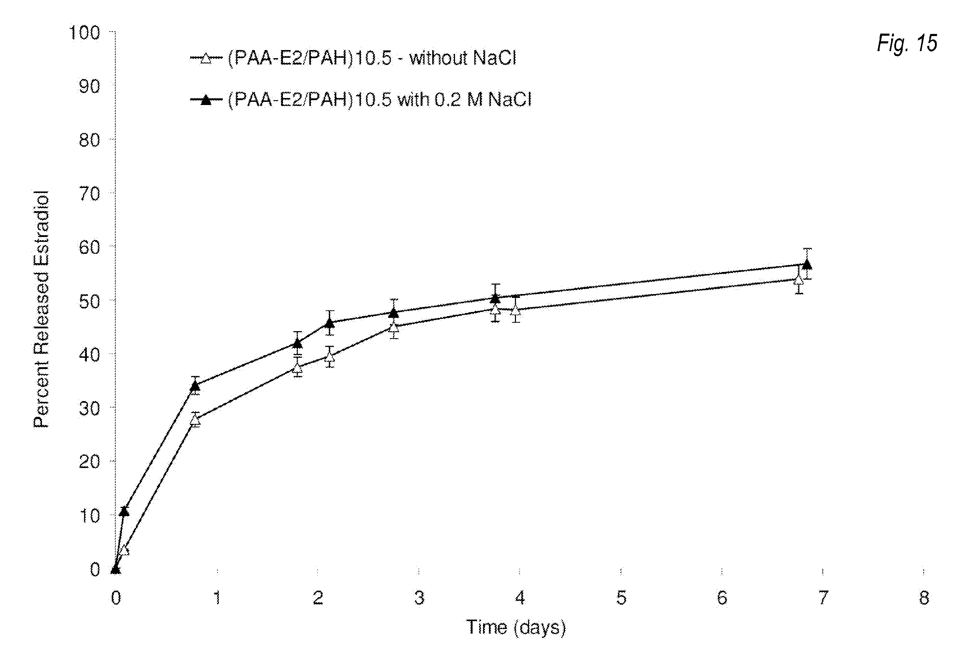

PEM were constructed with PAA-E2 and PAH at pH 5.6 (50 mM acetate buffer solution), 0.2 mg/ml polyelectrolyte concentration with 0.2 M NaCl. The build-up was monitored with UV spectroscopy (Figure 13) and QCM-D (Figure 14) up to 21 polyelectrolyte layers. The coating exhibits an exponential growth. The drug release is shown in Figure 15.

### Example 5:

PEM were constructed with PAA-E2 and PAH at pH 5.6 (50 mM acetate buffer solution), 0.2 mg/ml polyelectrolyte concentration without additional salt. The build-up was monitored with UV spectroscopy (Figure 13) up to 21 polyelectrolyte layers. The coating exhibits an exponential growth. The drug release is shown in Figure 15.

### Example 6:

PEM were constructed with PG75-Ptx and PLL at pH 7.6 (50 mM phosphate buffer solution), 0.2 mg/ml polyelectrolyte concentration with 0.2 M NaCl. The build-up was monitored with UV spectroscopy (Figure 9) up to 21 polyelectrolyte layers. Compared to the build-up of PEM without additional salt a lower film growth was observed. The coating exhibits an exponential growth. The drug release is shown in Figures 11 and 12.

While preferred embodiments have been illustrated and described in detail in the drawings and foregoing description, the same is to be considered as illustrative and not restrictive in character, it being understood that only the preferred embodiment has been shown and described and that all changes and modifications that comes within the spirit of the invention both now or in the future are desired to be protected.

### Reference list

B. Thierry et al., "Bioactive Coatings of Endovascular Stents based on Polyelectrolyte Multilayers", Biomacromolecules 2003, 4, 1564-1571
B. Thierry et al., "Nanocoatings onto Arteries via Layer-by-Layer Deposition: Toward the in Vivo Repair of Damaged Blood Vessels", Journal of the American Chemical Society 2003, 125, 7494-7495.
B. Thierry et al., "Radionuclides-hyaluronan-conjugated thromboresistant coatings to prevent in-stent restenosis", Biomaterials 2004, 25, 3895-3905
B. Thierry et al., "Magnetic Resonance Signal-Enhancing Self-Assembled Coating for Endovascular Devices", Advanced Materials 2005, 17, No 7, 826-830
B. Thierry et al., "Delivery Plattform for Hydrophobic Drugs: Prodrug Approach Combined with Self-Assembled Multilayers", Journal of the American Chemical Society 2005, 127, 1626-1627
L. Huang and M. Yang, "Hemocompatibility of Layer-by-Layer Hyaluronic Acid/Heparin Nanostructures Coating on Stainless Steel for Cardiovascular Stents and its Use for Drug Delivery", Journal of Nanoscience and Nanotechnology, 2006, 6, 3163-3170
C. Jewell et al., "Release of Plasmid DNA from Intravascular Stents Coated with Ultrathin Multilayered Polyelectrolyte Films", Biomacromolecules 2006, 7, 2483-2491.
M. Zovko et al., "Macromolecular prodrugs. XI. Synthesis and characterisation of polymer-estradiol conjugate", International Journal of Pharmaceutics 2004, 285, 35-41
R. Schoenmakers et al., "The effect of the linker on the hydrolysis rate of drug-linked ester bonds", Journal of Controlled Release 2004, 95, 291-300
   US 6 497 729 B1
   US 6 515 017 B1
   US 2003/0161938 A1
   US 2004/0241202 A1
   US 2004/0254631 A1
   US 2005/0060028
   US 2007/0020469
   WO 2006/026187 A2

## Claims

1. A drug-releasing polyelectrolyte coating, comprising:
(a) at least two oppositely charged polyelectrolyte layers;
(b) at least one pharmaceutical active drug which is covalently coupled to polyelectrolytes of at least one of the polyelectrolyte layers;
(c) wherein the drug exhibits a release rate from the polyelectrolyte coating of less than 50% in 3 days.

2. The drug-releasing coating according to claim 1, wherein the drug exhibits a release rate from the polyelectrolyte coating of less than 50% in 5 days and particularly of less than 50% in 7 days.

3. The drug-releasing coating according to claim 1 or 2, wherein the pharmaceutical active drug is directly covalently coupled to the polyelectrolyte.

4. The drug-releasing coating according to any of the claims 1 to 3, wherein the pharmaceutical active drug is directly covalently coupled to the polyelectrolyte by a condensation reaction.

5. The drug-releasing coating according to any of the claims 1 to 4, wherein the pharmaceutical active drug is covalently coupled to the polyelectrolyte by at least one link selected from the group comprising esters, amides, carbamides, disulfides, and azomethines.

6. The drug-releasing coating according to any of the claims 1 to 5, wherein the polyelectrolyte comprises a biocompatible polymer.

7. The drug-releasing coating according to claim 6, wherein the polymer comprises a polycarboxylic acid.

8. The drug-releasing coating according to claim 6, wherein the polymer comprises a polyamine.

9. The drug-releasing coating according to any of the claims 1 to 8, wherein the pharmaceutical active drug is at least soluble in water.

10. The drug-releasing coating according to any of the claims 1 to 8, wherein the pharmaceutical active drug is only sparingly soluble in water.

11. The drug-releasing coating according to any of the claims 1 to 10 wherein the pharmaceutical active drug is selected from the group comprising analgetic, antiproliferativ, antithrombotic, anti-inflammatory, antimycotic, cytostatic, antibiotic, immunosuppressive substances as well as growth factors, hormones, glucocorticoids, steroids, non-steroidal substances, genetically or metabollically active substances for silencing and transfection, and any pharmaceutical acceptable derivative thereof.

12. The drug-releasing coating according to any of the claims 1 to 11, wherein the drug-releasing coating comprises a plurality of alternatingly charged polyelectrolyte layers.

13. The drug-releasing coating according to claim 12, wherein the pharmaceutical active drug is covalently coupled to the polyelectrolyte of at least two of the alternatingly charged polyelectrolyte layers.

14. An implantable device comprising
(a) a substrate having a surface; and
(b) a drug-releasing coating according to any of the claims 1 to 13, the drug-releasing coating covers at least a portion of the surface.

15. The implantable device according to claim 14, wherein the implantable device is selected from the group comprising stents, catheters, pacemakers, and artificial vessels of permanent or transient types.

16. The implantable device according to claim 14, wherein the substrate comprises a biodegradable matrix.

17. The implantable device according to claim 16, wherein the biodegradable matrix comprises at least one material selected from the group containing polyanhydrides, polyesters, polypetides, calcium phosphate, nucleic acids, hydroxyapatite, polylactides, poly(lactide-co-glucolide), cationic and anionic polyelectrolytes, biopolymers, and mixtures thereof.

18. A method for forming a drug-releasing polyelectrolyte coating, comprising the steps of:
(a) providing a substrate comprising a surface;
(a) depositing at least two oppositely charged polyelectrolyte layers on at least a portion of the surface to form a polyelectrolyte multilayer on the surface, at least one of the polyelectrolyte layers comprising at least one pharmaceutical active drug which is covalently coupled to polyelectrolytes of this layer;
(c) wherein the pharmaceutical active drug is coupled to the polyelectrolyte such that it exhibits a release rate from the polyelectrolyte layer of less than 50% in 3 days.

19. The method according to claim 18, further comprising the step of directly covalently coupling the pharmaceutical active drug to the polyelectrolyte before depositing the polyelectrolyte layer.

20. The method according to claim 18 or 19, wherein the pharmaceutical active drug is directly covalently coupled to the polyelectrolyte by a condensation reaction.

21. The method according to any of the claims 18 to 20, wherein the pharmaceutical active drug is coupled to the polyelectrolyte by forming at least one link between the pharmaceutical active drug and the polyelectrolyte, the link being selected from the group containing ester link, amide link and Schiff base link.

22. The method according to any of the claims 18 to 21, wherein the polyelectrolyte comprises a biocompatible polymer.

23. The method according to any of the claims 18 to 22, wherein the deposition of a polyelectrolyte layer comprises:
- depositing polyelectrolytes or drug-conjugated polyelectrolytes of a given charge;
- at least partially drying the deposited polyelectrolytes or drug-conjugated polyelectrolytes; and
- depositing polyelectrolytes or drug-conjugated polyelectrolytes of the given charge.

24. The method according to any of the claims 18 to 23, wherein at least one intermediate step is carried out between two consecutive deposition steps, wherein the intermediate step is selected from the group comprising washing, rinsing or drying.

25. The method according to any of the claims 18 to 24, wherein the drug-coupled polyelectrolyte is deposited on the surface in a hypotonic solution.

26. The method according to any of the claims 11 to 25, wherein the drug-coupled polyelectrolyte is deposited on an implantable device.

27. A method for forming a drug-releasing polyelectrolyte coating, comprising the steps of:
(a) providing a substrate comprising a surface;
(a) depositing at least two oppositely charged polyelectrolyte layers on at least a portion of the surface to form a polyelectrolyte multilayer on the surface, at least one of the polyelectrolyte layers comprising at least one pharmaceutical active drug which is covalently coupled to a polyelectrolyte of this layer;
(b) wherein the drug-coupled polyelectrolyte is deposited on the surface in a hypotonic solution.

28. The method according to claim 27, wherein the hypotonic solution has a molarity of salt of less than 0.1 M and particularly of less than 0.05 M.

29. The method according to claim 26 or 27, wherein the hypotonic solution is salt-free
